(19) Europäisches Patentamt / European Patent Office / Office européen des brevets

(11) EP 2 029 521 B1

(12) **EUROPÄISCHE PATENTSCHRIFT**

(45) Veröffentlichungstag und Bekanntmachung des Hinweises auf die Patenterteilung:
**11.07.2012 Patentblatt 2012/28**

(21) Anmeldenummer: **07729483.3**

(22) Anmeldetag: **24.05.2007**

(51) Int Cl.:
***C07C 209/26*** (2006.01) ***C07D 295/02*** (2006.01)

(86) Internationale Anmeldenummer:
**PCT/EP2007/055054**

(87) Internationale Veröffentlichungsnummer:
**WO 2007/137990 (06.12.2007 Gazette 2007/49)**

(54) **VERFAHREN ZUR HERSTELLUNG EINES AMINS**

PROCESS FOR THE PREPARATION OF AN AMINE

PROCÉDÉ DE PRODUCTION D'UNE AMINE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **31.05.2006 EP 06114777**

(43) Veröffentlichungstag der Anmeldung:
**04.03.2009 Patentblatt 2009/10**

(73) Patentinhaber: **BASF SE**
**67056 Ludwigshafen (DE)**

(72) Erfinder:
- **EBERHARDT, Jan**
  **68167 Mannheim (DE)**
- **MEISSNER, Harald**
  **67454 Hassloch (DE)**
- **HOFFER, Bram, Willem**
  **69120 Heidelberg (DE)**
- **MELDER, Johann-Peter**
  **67459 Böhl-Iggelheim (DE)**
- **SCHWAB, Ekkehard**
  **67434 Neustadt (DE)**

(56) Entgegenhaltungen:
**EP-A1- 1 020 424** **JP-A- 2 180 854**
**JP-A- 10 081 650**

EP 2 029 521 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Herstellung eines Amins durch Umsetzung eines Aldehyds und/oder Ketons mit Wasserstoff und einer Stickstoffverbindung, ausgewählt aus der Gruppe primärer und sekundärer Amine, in Gegenwart eines Heterogenkatalysators.

Die Verfahrensprodukte finden u.a. Verwendung als Zwischenprodukte bei der Herstellung von Kraftstoffadditiven (US-A-3,275,554; DE-A-21 25 039 und DE-A-36 11 230), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmittein, Farbstoffen, Vulkanisationsbeschleunigern und/oder Emulgatoren.

Zur Herstellung eines Amins durch Umsetzung eines Aldehyds oder Ketons mit Wasserstoff und einer Stickstoffverbindung sind z.B. Hochdruckverfahren bekannt. Hier erfolgt die hydrierende Aminierung am Katalysatorfestbett, wobei z.B. Metallkatalysatoren mit Ni, Pd, Pt, Promotoren auf einem Träger eingesetzt werden.

DE-A-211 82 83 (BASF AG) betrifft ein Verfahren zur Herstellung sekundärer oder tertiärer aliphatischer oder cycloaliphatischer Amine unter Einsatz eines Pd/Ag-Festbettkatalysators. Trägermaterial ist insbesondere $SiO_2$.

EP-A1-7093 (BASF AG) betrifft die Herstellung von N-Aralkyl-2,6-dimethylmorpholinen, wie z.B. Fenpropimorph, an Pd/Ag-Festbettkatalysatoren.

EP-A1-1 020 424 (BASF AG) betrifft die Herstellung von N-Ethyl-diisopropylamin an Festbettkatalysatoren mit bestimmten oxidischen Trägermaterial (wie z.B. $ZrO_2$).

Die WO-Patentanmeldung 2007/107477 A1 mit Priorität vom 21.03.06 (BASF AG) betrifft ein Verfahren zur Herstellung eines Amins durch Umsetzung eines Aldehyds und/oder Ketons mit Wasserstoff und einer Stickstoffverbindung in Gegenwart eines Heterogenkatalysators, wobei es sich bei dem Festbettkatalysator um einen bestimmten Schalenkatalysator handelt.

Zur Herstellung eines Amins durch hydrierende Aminierung sind auch Niederdruckverfahren bekannt. Hier kommen z.B. Edelmetallkatalysatoren in Suspensionsfahrweise zum Einsatz. Z. B. zur Herstellung von Dimethylcyclohexylamin (DMCHA) an Pd/C: US-A-4,521,624 (1985) (Druckbereich 3,4-40 bar, Temperaturen 70 bis 135°C) und CN-A-1 092 061 (1994) (Druckbereich 10-50 bar).

JP 10081650 A2, erteilt als JP 2851274 B2 (Koei Chem.), sowie JP 02180854 A2, erteilt als JP 2740828 B2 (Koei Chem.), beschreiben insbesondere die Herstellung von Ethyldiisopropylamin aus Acetaldehyd und Düsopropylamin oder aus Aceton und Ethylamin an Pd/C-Suspensionskatalysatoren in semibatch-Fahrweise bei 20 - 200°C und bevorzugt 5,07 - 60,8 bar (5 -60 atm). Diese Schriften lehren die Entfernung des Katalysators aus dem Reaktionsgefäß nach beendeter Umsetzung und lehren nichts zur Umsatzkontrolle des eingesetzten Amins.

EP-A-611 137 (Sumitomo Chem. Comp.) betrifft die reduktive Aminierung von zyklischen Ketonen, wobei in einer ersten Stufe eine entsprechende Imino-Verbindung hergestellt wird, die nachfolgend hydriert wird.

EP-A2-312 253 (Kao Corp.) beschreibt die Verwendung spezifischer Kupferkatalysatoren bei der Herstellung von n-substituierten Aminen aus Alkoholen oder Aldehyden.

Der vorliegenden Erfindung lag die Aufgabe zugrunde, unter Überwindung eines Nachteils oder mehrerer Nachteile des Stands der Technik, ein verbessertes wirtschaftliches Verfahren zur Herstellung eines Amins aufzufinden. Insbesondere sollte das Verfahren einen Katalysator hoher Aktivität beinhalten, der durch geschickte Wahl der Verfahrensbedingungen eine hohe Selektivität in der Reaktion zeigt und mehrfach wiedereinsetzbar ist. Das Verfahrensprodukt sollte in hohen Ausbeuten, insbesondere auch Raum-Zeit-Ausbeuten (RZA), anfallen.

Demgemäß wurde ein Verfahren zur Herstellung eines Amins durch Umsetzung eines Aldehyds und/oder Ketons mit Wasserstoff und einer Stickstoffverbindung, ausgewählt aus der Gruppe primärer und sekundärer Amine, in Gegenwart eines Heterogenkatalysators gefunden, welches dadurch gekennzeichnet ist, dass die Umsetzung unter Einsatz eines Suspensionskatalysators als Heterogenkatalysator erfolgt und

in semibatch-Fahrweise durchgeführt wird, bei der die Stickstoffverbindung als ein Reaktionspartner im Reaktionsgefäß vorgelegt und der Aldehyd und/oder das Keton als der andere Reaktionspartner im Reaktionsverlauf zugegeben wird, und im Reaktionsverlauf in Abhängigkeit vom erreichten Umsatz der Stickstoffverbindung der Aldehyd und/oder das Keton portionsweise oder kontinuierlich zum Reaktionsgemisch gegeben wird, bis ein Umsatz der Stickstoffverbindung von mindestens 95 % resultiert,

und der Katalysator ganz oder teilweise nach der Reaktionspartie für die nächste Reaktionspartie zum erneuten Einsatz im Reaktionsgefäß verbleibt und wieder eingesetzt wird, wobei unter "teilweisem Verbleib des Katalysators im Reaktionsgefäß" zu verstehen ist, dass mindestens 30 Gew.-% des ursprünglich eingesetzten Katalysators nach

Durchführung der Umsetzung und Abtrennung der organischen Reaktionsprodukte im Reaktionsgefäß verbleiben.

Zu den Vorteilen des erfindungsgemäßen Verfahrens zählt u.a., dass in der semibatch-Fahrweise im Reaktionsverlauf nur geringe Konzentrationen von nicht-umgesetztem Aldehyd/Keton vorliegen und deshalb nur kleine Mengen an Nebenprodukten, z.B. aus der basisch katalysierten Aldolreaktion, gebildet werden. Dank der Bestimmung des

Umsatzes im Versuchsverlauf und gezielten Zugabe von Aldehyd/Keton bis zum Erreichen eines hohen spezifischen Mindestumsatzes kann auf eine Rückführung von nicht-umgesetzter Stickstoffkomponente verzichtet werden, zumal die destillative Trennung von nicht-umgesetzten Einsatzstoffen, Wasser und ggf. Nebenprodukten oftmals schwierig ist.

Das Anfahren der Reaktion erfolgt bevorzugt in folgender Reihenfolge:

(a) der Reaktor wird mit der umzusetzenden Stickstoffverbindung und dem Katalysator beschickt,
(b) der Reaktor wird mit Stickstoff gespült,
(c) ein Wasserstoffdruck wird eingestellt, der geringer als der spätere Reaktionsdruck ist, z. B. wird ein Druck von 10 bar eingestellt, wenn die Reaktion bei einem Enddruck von 50 bar erfolgen soll,
(d) der Reaktor wird auf Reaktionstemperatur aufgeheizt,
(e) der Wasserstoffdruck wird auf Reaktionsdruck erhöht,
(f) die Carbonylverbindung wird portionsweise oder kontinuierlich zum Reaktionsgemisch gegeben.

Der Aldehyd und/oder das Keton wird im erfindungsgemäßen Verfahren bevorzugt bei Reaktionstemperatur und -druck zu der vorgelegten Stickstoffverbindung mit Katalysator gegeben.

Die Zugabe erfolgt, bevorzugt innerhalb von 0,5 bis 24 Stunden, weiter bevorzugt innerhalb von 1 bis 15 Stunden, kontiniuierlich oder portionsweise. Zu aufeinanderfolgenden Zeitpunkten, z.B. alle 30 Min. oder alle 15 Min., oder kontinuierlich, z.B. per online-Gaschromatographie oder online-Spektroskopie, wird der Umsatz geprüft. Der Umsatz wird z.B. überprüft, indem eine kleine Probemenge aus dem Reaktor entnommen wird und die Zusammensetzung analysiert wird, beispielweise mittels Gaschromatographie. Der Umsatz (U) errechnet sich aus dem Quotienten der Mengenanteile der aus der eingesetzten Stickstoffverbindung gebildeten Produkte und der Summe der Mengenanteile der aus der Stickstoffverbindung gebildeten Produkte und nicht-umgesetzter Stickstoffverbindung gemäß folgender Formel:

$$U = \frac{n_2}{n_2 + n_1} * 100\%$$

mit $n_2$ = Stoffmenge der aus der Stickstoffverbindung gebildeten Produkte und $n_1$ = Stoffmenge an nicht-umgesetzter Stickstoffverbindung

Der Aldehyd und/oder das Keton wird solange dosiert, bis ein Umsatz von mindestens 95 %, bevorzugt mindestens 96 %, besonders bevorzugt mindestens 97 %, z.B. 97,5 bis 99,8 %, jeweils bezüglich eingesetzter Stickstoffverbindung, erreicht ist.

Das bedeutet, dass je nach Selektivität der Reaktion der Aldehyd und/oder das Keton äquimolar oder im Überschuss eingesetzt wird. Das molare Einsatzstoffverhältnis von Aldehyd und/oder Keton zu eingesetzter Stickstoffverbindung liegt bevorzugt im Bereich von 1,0 bis 3,5, besonders im Bereich von 1,01 bis 1,50, ganz besonders im Bereich von 1,02 bis 1,25.

In einer besonders bevorzugten Ausführungsform der Erfindung wird die Zugabegeschwindigkeit des Aldehyds und/oder des Ketons so gewählt, dass die jeweils gewünschte Maximaltemperatur (bevorzugt im Bereich von 70 bis 180 °C) der Reaktion nicht überschritten wird. (Geschwindigkeit der Zugabe z.B. in Mol Aldehyd und/oder Keton pro 30 Min.).

Bei Zugabe von Aldehyd bzw. Keton zur Stickstoffverbindung erfolgen Reaktionen, in denen Wärme freigesetzt wird: Das Zwischenprodukt Imin (wenn es sich bei der vorgelegten Stickstoffverbindung um ein primäres Amin handelt) bzw. Enamin (wenn es sich bei der vorgelegten Stickstoffverbindung um ein sekundäres Amin handelt) wird in einer exothermen Reaktion unter Wasserabspaltung gebildet. Das Zwischenprodukt wird dann in einer weiteren exothermen Reaktion in Anwesenheit von Wasserstoff und dem Hydrierkatalysator zum Produkt umgesetzt (Hydrierung der Doppelbindung). Die Reaktionstemperatur kann auf eine sehr einfache Weise kontrolliert und begrenzt werden, indem die Zugabe des Aldehyds und/oder Ketons vor Erreichen einer zuvor festgelegten Maximaltemperatur verlangsamt oder kurzzeitig unterbrochen wird, bis die Reaktionsmischung durch die Wärmeabstrahlung des Reaktionsgefäßes oder unter Anwendung einer externen Kühlung wieder erniedrigt wurde.

Nach Abschluss der Reaktion lässt man den Katalysator im Reaktionsgefäß absetzen, wobei sich - je nach hergestelltem Amin - ggf. zwei Phasen (eine organische und eine wässrige Phase) im Reaktionsgemisch ausbilden. Die organische Phase und bevorzugt, falls gegeben, ein Teil der wässrigen Phase, wird dann, bevorzugt über ein Filter, aus dem Reaktor gefahren.

Überraschenderweise wurde gefunden, dass der Katalysator eine sehr hohe Katalyseaktivität behält, wenn er nach Abschluss der Reaktion - bevorzugt, falls gegeben, zusammen mit der gebildeten wässrigen Phase oder einem Teil der gebildeten wässrigen Phase des Reaktionsaustrages - ganz oder zumindest teilweise im Reaktionsgefäß (Reaktor) verbleibt. Der nachfolgende Reaktionsansatz kann dann, bevorzugt ohne weitere Katalysatorspülung oder anderweitige Katalysatorreaktivierung (Regenerierung), durchgeführt werden, indem erfindungsgemäß wieder die Stickstoffverbindung in den Reaktor gegeben wird, Wasserstoff zum Gemisch gegeben wird, die Mischung auf Reaktionstemperatur erwärmt wird und dann der Aldehyd und/oder das Keton zugegeben wird. In einer besonderen Ausführungsform der Erfindung wird im nachfolgenden Reaktionsansatz (= nachfolgende Reaktionspartie) die Stickstoffverbindung in der Art und Weise in den Reaktor gegeben, dass der o.g. Filter mit der gleichen Stickstoffverbindung gespült wird, sodass eventuell im Filter befindliche Katalysatorpartikel wieder in den Reaktor überführt werden.

Durch den teilweisen Verbleib der wässrigen Phase im Reaktionsgefäß wird der Wassergehalt im Reaktionsgemisch in der nachfolgenden Reaktionspartie erhöht. Überraschenderweise wurde gefunden, dass die Anwesenheit der größeren Wassermenge sich insgesamt nicht nachteilig auf die Reaktion auswirkt, obwohl die vorgelagerte Gleichgewichtsreaktion (Bildung des Enamins bzw. Imins unter Wasserabspaltung) durch die erhöhten Wasserkonzentrationen negativ beeinflusst werden. Der Vorteil, dass der Katalysator eine hohe Aktivität behält, überwiegt den Nachteil bezüglich der erhöhten Wasserkonzentration, sodass die beschriebene Vorgehensweise bei der Katalysatorrückführung insgesamt vorteilhaft ist.

Unter "teilweisem Verbleib des Katalysators im Reaktionsgefäß" ist insbesondere zu verstehen, dass mindestens 50 Gew.-%, weiter besonders mindestens 70 Gew.-%, ganz besonders mindestens 90 Gew.-%, des urspünglich eingesetzten Katalysators nach Durchführung der Umsetzung und Abtrennung der organischen Reaktionsprodukte im Reaktionsgefäß verbleiben.

Unter "teilweisem Verbleib der wässrigen Phase im Reaktionsgefäß" ist insbesondere zu verstehen, dass mindestens 20 Gew.-%, besonders mindestens 30 Gew.-%, weiter besonders mindestens 40 Gew.-%, ganz besonders mindestens 50 Gew.-%, der nach Durchführung der Umsetzung gebildeten wässrigen Phase im Reaktionsgefäß verbleiben.

Ein weiterer Vorteil des erfindungsgemäßen Verfahrens ist damit ein sehr einfacher und schneller Ablauf der einzelnen Partien nacheinander, ohne aufwändige Katalysatorausschleusung, bei der Teile an Katalysator mit Luft in Kontakt kommen können oder auf dem Filter verbleiben (Stoffverluste). Ohne das aufwändige Katalysatorhandling ist das Verfahren sicherer betreibbar, und gleichzeitig werden die Herstellkosten verringert, indem der Katalysatorverbrauch minimiert wird, das Verfahren schnell (erhöhte Kapazität in bestehenden Anlagen) und mit kleinerem Arbeitsaufwand (geringerer Personalaufwand) durchführbar ist.

Durch das erfindungsgemäße Verfahren ist es ebenfalls ermöglicht, die Reaktion mit einer konstanten Katalyseaktivität durchzuführen, indem z. B. bei nachlassender Reaktionsgeschwindigkeit/verlängerten Batchzeiten beispielsweise kleine Mengen an frischem Katalysator zugegeben werden, wodurch eine absolut gleichbleibende Produktqualität im zeitlichen Verlauf ermöglicht wird. Die Aufarbeitung und Reinigung des Rohproduktes wird dadurch entscheidend vereinfacht. Im Verlauf einer Kampagne wird in allen Partien durchweg ein weitgehend identischer Rohaustrag aufgearbeitet. Die Anteile an Wertprodukt (Verfahrensprodukt) und/oder Nebenprodukten sind weitgehend gleichbleibend. Die Trennaufgabe in der nachfolgenden Aufarbeitung, z.B. Destillation, ändert sich im zeitlichen Verlauf nicht oder nur unwesentlich.

Im Suspensionskatalysator des erfindungsgemäßen Verfahrens sind die katalytisch aktiven Metalle oder die Metalle in deren Verbindungen bevorzugt ausgewählt aus den Elementen der Gruppen 8 und/oder 9 und/oder 10 und/ oder 11 des Periodensystems der Elemente (IUPAC notation 1985).

Das erfindungsgemäße Verfahren wird bevorzugt in Gegenwart eines geträgerten Übergangsmetallkatalysators als Suspensionskatalysator durchgeführt. Erfindungsgemäß bevorzugte Übergangsmetallkatalysatoren sind insbesondere solche, die als Aktivkomponenten eines oder mehrere Metalle ausgewählt aus der Gruppe der Metalle Pd, Pt, Ag, Ru, Rh, Ni und Pd enthalten. Besonders bevorzugt ist Pd als zumindest eine, insbesondere einzige, Aktivkomponente. (Aktivkomponente = katalytisch aktive Komponente).

Als Trägermaterialien der erfindungsgemäß einzusetzenden Katalysatoren kommen beispielsweise Aktivkohle, Aluminiumoxid, Kieselgel, $CaCO_3$, $BaSO_4$, $ZrO_2$, $TiO_2$, bevorzugt Aktivkohle und Aluminiumoxid und besonders bevorzugt Aktivkohle in Betracht.

Ein im Rahmen der vorliegenden Erfindung besonders bevorzugter Katalysator ist Pd auf Aktivkohle (Pd/C).

Die genannten Katalysatoren weisen vorteilhafterweise Metallgehalte, insbesondere Edelmetallgehalte, von 0,1 bis 25 Gew.-%, bevorzugt von 0,5 bis 15 Gew.-% und besonders bevorzugt von 4 bis 11 Gew.-% (jeweils bezogen auf das reduzierte Metall bzw. die reduzierten Metalle des fertigen Katalysators und bezogen auf die Gesamtmasse des trockenen Katalysators) auf.

Derartige Katalysatoren sind kommerziell zugänglich und beispielsweise unter den Bezeichnungen Degussa E1002, Degussa E101, Degussa E105, Degussa E106, Engelhard C3630, Heraeus K201, Heraeus K202, Heraeus K203, Heraeus K204, Heraeus K219 erhältlich.

Der gewählte Katalysator wird vorteilhaft in einer solchen Menge eingesetzt, dass die Menge an Katalysator (wasserfrei gerechnet) bezogen auf die Menge an umzusetzendem primären oder sekundären Amin im Bereich von 0,1 bis 20,0 Gew.-%, besonders im Bereich von 0,5 bis 5,0 Gew.-%, beträgt.

Der Suspensionskatalysator weist besonders einen Wassergehalt im Bereich von 1 bis 70 Gew.-%, weiter besonders

im Bereich von 30 bis 60 Gew.-%, auf.

In einer bevorzugten Ausführungsform wird die Umsetzung im erfindungsgemäßen Verfahren ohne Zusatz von Promotoren im Katalysator, wie z.B. Zink-Dotierungen, oder Hilfsstoffen, wie z.B. Kohlenmonoxid, durchführt.

Durch das erfindungsgemäße Verfahren können Aldehyde und Ketone mit hoher Selektivität und hoher Ausbeute in die entsprechenden sekundären und tertiären Amine überführt werden.

Die Aminierung des Aldehyds und/oder Ketons erfolgt bevorzugt in der Flüssigphase.

Gemäß einer Ausführungsform der Erfindung wird die Umsetzung in der Flüssigphase oder in einer gemischten Flüssig-/Gasphase mit mindestens 50 Gew.-% des Reaktionsgemisches in der Flüssigphase durchgeführt.

Als Reaktoren können z.B. Rührkessel, Autoklaven, Schlaufenreaktoren oder gepackte Blasensäulen verwendet werden. Bevorzugter Reaktor ist ein Rührkessel.

Um die Ausbildung einer Flüssigphase zu gewährleisten, müssen geeignete Temperatur- und Druckparameter in den o.g. Bereichen gewählt werden, was abhängig vom jeweilig eingesetzten Stoffgemisch ist.

Das erfindungsgemäße Verfahren wird bevorzugt bei einem Absolutdruck (= Reaktionsdruck) im Bereich von 1 bis 120 bar, bevorzugt 5 bis 100 bar, besonders bevorzugt 10 bis 80 bar, durchgeführt. Der Druck im Reaktor, welcher sich aus der Summe der Partialdrücke des Aminierungsmittels, der Aldehyd- und/oder Keton-Komponente und der gebildeten Reaktionsprodukte bei den angegebenen Temperaturen ergibt, wird zweckmäßigerweise durch Aufpressen von Wasserstoff auf den gewünschten Reaktionsdruck erhöht.

Das erfindungsgemäße Verfahren der Aldehyd- und/oder Ketonaminierung wird bevorzugt bei einer Temperatur im Bereich von 15 bis 180 °C, bevorzugt 30 bis 170 °C, besonders bevorzugt 70 bis 160 °C, durchgeführt.

Bevorzugt wird eine Abgasmenge von 0,1 bis 400 Normkubikmeter/h/(Liter Reaktionsvolumen), insbesondere 1 bis 20 Normkubikmeter/h/(Liter Reaktionsvolumen), gefahren.

Die Anwendung höherer Temperaturen, höherer Gesamtdrücke und kleinerer Katalysatormengen ist möglich. Aus dem Reaktionsaustrag werden, nachdem dieser zweckmäßigerweise entspannt worden ist, der überschüssige Wasserstoff und das gegebenenfalls noch in Spuren vorhandene Aminierungsmittel entfernt und das erhaltene Reaktionsrohprodukt gereinigt, z.B. durch eine fraktionierende Rektifikation. Geeignete Aufarbeitungsverfahren sind z.B. in EP-A-1 312 600 und EP-A-1 312 599 (beide BASF AG) beschrieben.

Mit dem erfindungsgemäßen Verfahren herstellbar sind z.B. Amine der Formel I

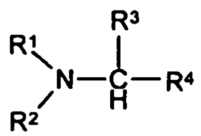

(I),

in der

| | |
|---|---|
| $R^1$, $R^2$ | Wasserstoff (H), Alkyl, wie $C_{1-20}$-Alkyl , Cycloalkyl, wie $C_{3-12}$-Cycloalkyl, Alkoxyalkyl, wie $C_{2-30}$-Alkoxyalkyl, Dialkylaminoalkyl, wie $C_{3-30}$-Dialkylaminoalkyl, Aryl, Aralkyl, wie $C_{7-20}$-Aralkyl, und Alkylaryl, wie $C_{7-20}$-Alkylaryl, oder gemeinsam $-(CH_2)_j-X-(CH_2)_k-$, ($R^1$ und $R^2$ sind nicht beide gleichzeitig H), |
| $R^3$, $R^4$ | Wasserstoff (H), Alkyl, wie $C_{1-20}$-Alkyl, Cycloalkyl, wie $C_{3-12}$-Cycloalkyl, Hydroxyalkyl, wie $C_{1-20}$-Hydroxyalkyl, Aminoalkyl, wie $C_{1-20}$-Aminoalkyl, Hydroxyalkylaminoalkyl, wie $C_{2-20}$- Hydroxyalkylaminoalkyl, Alkoxyalkyl, wie $C_{2-30}$-Alkoxyalkyl, Dialkylaminoalkyl, wie $C_{3-30}$-Dialkylaminoalkyl, Alkylaminoalkyl, wie $C_{2-30}$-Alkylaminoalkyl, $R^5-(OCR^6R^7CR^8R^9)_n-(OCR^6R^7)$, Aryl, Heteroaryl, Aralkyl, wie $C_{7-20}$-Aralkyl, Heteroarylalkyl, wie $C_{4-20}$-Heteroarylalkyl, Alkylaryl, wie $C_{7-20}$-Alkylaryl, Alkylheteroaryl, wie $C_{4-20}$-Alkylheteroaryl, und $Y-(CH_2)_m-NR^5-(CH_2)_q$ oder gemeinsam $-(CH_2)_l-X-(CH_2)_m-$ oder |
| $R^2$ und $R^4$ | gemeinsam $-(CH_2)_l-X-(CH_2)_m-$, |
| $R^5$, $R^{10}$ | Wasserstoff (H), Alkyl, wie $C_{1-4}$Alkyl, Alkylphenyl, wie $C_{7-40}$-Alkylphenyl, |
| $R^6$, $R^7$, $R^8$, $R^9$ | Wasserstoff (H), Methyl oder Ethyl, |
| X | $CH_2$, $CHR^5$, Sauerstoff (O), Schwefel (S) oder $NR^5$, |
| Y | $N(R^{10})_2$, Hydroxy, $C_{2-20}$-Alkylaminoalkyl oder $C_{3-20}$-Dialkylaminoalkyl, |
| n | eine ganze Zahl von 1 bis 30 und |

j, k, l, m, q eine ganze Zahl von 1 bis 4,

bedeuten.

Das erfindungsgemäße Verfahren findet daher bevorzugt zur Herstellung eines Amins I Anwendung, indem man einen Aldehyd und/oder ein Keton der Formel VI bzw. VII

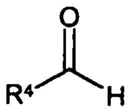 (VI) 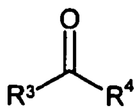 (VII)

mit einer Stickstoffverbindung der Formel III

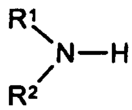 (III),

wobei $R^1$, $R^2$, $R^3$ und $R^4$ die oben genannten Bedeutungen haben, umsetzt.

Wie aus den Definitionen für die Reste $R^2$ und $R^4$ hervorgeht, kann die Umsetzung auch intramolekular in einem entsprechenden Aminoketon oder Aminoaldehyd erfolgen.

Zur Herstellung des Amins I wird demnach rein formal ein Wasserstoffatom der Stickstoffverbindung III durch den Rest $R^4(R^3)CH$- unter Freisetzung von einem Moläquivalent Wasser ersetzt.

Die Substituenten $R^1$ bis $R^{10}$, die Variablen X, Y und die Indizes j, k, l, m, n und q in den Verbindungen I,III, VI und VII haben unabhängig voneinander folgende Bedeutungen:

$R^1$, $R^2$, $R^3$, $R^4$, $R^5$, $R^6$, $R^7$, $R^8$, $R^9$, $R^{10}$:

- Wasserstoff (H), ($R^1$ und $R^2$ sind nicht beide gleichzeitig H),

R3, $R^4$:

- Alkyl, wie $C_{1-20}$-Alkyl, bevorzugt $C_{1-14}$Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl,

- Hydroxyalkyl, wie $C_{1-20}$-Hydroxyalkyl, bevorzugt $C_{1-8}$-Hydroxyalkyl, besonders bevorzugt $C_{1-4}$-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-(Hydroxymethyl)ethyl,

- Aminoalkyl, wie $C_{1-20}$-Aminoalkyl, bevorzugt $C_{1-8}$-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(2-Aminoethyl)-2-aminoethyl und N-(2-Aminoethyl)aminomethyl,

- Hydroxyalkylaminoalkyl, wie $C_{2-20}$-Hydroxyalkylaminoalkyl, bevorzugt $C_{3-8}$-Hydroxyalkylaminoalkyl, wie (2-Hydroxyethylamino)methyl, 2-(2-Hydroxyethyl-amino)ethyl und 3-(2-Hydroxyethylamino)propyl,

- $R^5$-$(OCR^6R^7CR^8R^9)_n$-$(OCR^6R^7)$, bevorzugt $R^5$-$(OCHR^7CHR^9)_n$-$(OCR^6R^7)$, besonders bevorzugt $R^5$-$(OCH_2CHR^9)_n$-$(OCR^6R^7)$,

- Alkylaminoalkyl, wie $C_{2-30}$-Alkylaminoalkyl, bevorzugt $C_{2-20}$-Alkylaminoalkyl, besonders bevorzugt $C_{2-8}$-Alkylaminoalkyl, wie Methylaminomethyl, 2-Methylaminoethyl, Ethylaminomethyl, 2-Ethylaminoethyl und 2-(iso-Propylamino)ethyl, $(R^5)HN$-$(CH_2)_q$,

- Y-$(CH_2)_m$-$NR^5$-$(CH_2)_q$,

- Heteroarylalkyl, wie $C_{4-20}$-Heteroarylalkyl, wie Pyrid-2-yl-methyl, Furan-2-yl-methyl, Pyrrol-3-yl-methyl und Imidazol-2-yl-methyl,

- Alkylheteroaryl, wie $C_{4-20}$-Alkylheteroaryl, wie 2-Methyl-3-pyridinyl, 4,5-Dimethyl-imidazol-2-yl, 3-Methyl-2-furanyl und 5-Methyl-2-pyrazinyl,

- Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,

$R^1$, $R^2$, $R^3$, $R^4$:

- Cycloalkyl, wie $C_{3-12}$-Cycloalkyl, bevorzugt $C_{3-8}$-Cycloalkyl, wie Cyclopropyl, Cyclobutyl, Cyclopentyl, Cyclohexyl, Cycloheptyl und Cyclooctyl, besonders bevorzugt Cyclopentyl und Cyclohexyl,

- Alkoxyalkyl, wie $C_{2-30}$-Alkoxyalkyl, bevorzugt $C_{2-20}$-Alkoxyalkyl, besonders bevorzugt $C_{2-8}$-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxyethyl, besonders bevorzugt $C_{2-4}$-Alkoxyalkyl,

- Dialkylaminoalkyl, wie $C_{3-30}$-Dialkylaminoalkyl, bevorzugt $C_{3-20}$-Dialkylaminoalkyl, besonders bevorzugt $C_{3-10}$-Dialkylaminoalkyl, wie N,N-Dimethylaminomethyl, (N,N-Dibutylamino)methyl, 2-(N,N-Dimethylamino)ethyl, 2-(N,N-Diethylamino)ethyl, 2-(N,N-Dibutylamino)ethyl, 2-(N,N-Di-n-propylamino)ethyl und 2-(N,N-Di-isopropylamino)ethyl, 3-(N,N-Dimethylamino)propyl, $(R^5)_2N-(CH_2)_q$,

- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,

- Alkylaryl, wie $C_{7-20}$-Alkylaryl, bevorzugt $C_{7-12}$-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,

- Aralkyl, wie $C_{7-20}$-Aralkyl, bevorzugt $C_{7-12}$-Phenylalkyl, wie Benzyl, p-Methoxybenzyl, 3,4-Dimethoxybenzyl, 1-Phenethyl, 2-Phenethyl, 1-Phenyl-propyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,

- $R^3$ und $R^4$ oder $R^2$ und $R^4$ gemeinsam eine $-(CH_2)_l-X-(CH_2)_m-$ Gruppe, wie $-(CH_2)_3-$, $-(CH_2)_4-$, $-(CH_2)_5-$, $-(CH_2)_6-$, $-(CH_2)_7-$, $-(CH_2)-O-(CH_2)_2-$, $-(CH_2)-NR^5-(CH_2)_2-$, $-(CH_2)-CHR^5-(CH_2)_2-$, $-(CH_2)_2-O-(CH_2)_2-$, $-(CH_2)_2-NR^5-(CH_2)_2-$, $-(CH_2)_2-CHR^5-(CH_2)_2-$, $-CH_2-O-(CH_2)_3-$, $-CH_2-NR^5-(CH_2)_3-$, $-CH_2-CHR^5-(CH_2)_3-$,

$R^1$, $R^2$:

- Alkyl, wie $C_{1-20}$-Alkyl, bevorzugt $C_{1-8}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, 2-Ethylhexyl, besonders bevorzugt $C_{1-4}$-Alkyl, oder

- $R^1$ und $R^2$ gemeinsam eine $-(CH_2)_l-X-(CH_2)_k-$ Gruppe, wie $-(CH_2)_3-$, $-(CH_2)_4-$, $-(CH_2)_5-$, $-(CH_2)_6-$, $-(CH_2)_7-$, $-(CH_2)-O-(CH_2)_2-$, $-(CH_2)-NR^5-(CH_2)_2-$, $-(CH_2)-CHR^5-(CH_2)_2-$, $-(CH_2)_2-O-(CH_2)_2-$, $-(CH_2)_2-NR^5-(CH_2)_2-$, $-(CH_2)_2-CHR^5-(CH_2)_2-$, $-CH_2-O-(CH_2)_3-$, $-CH_2-NR^5-(CH_2)_3-$, $-CH_2-CHR^5-(CH_2)_3-$,

$R^5$, $R^{10}$:

- Alkyl, bevorzugt $C_{1-4}$-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,

- Alkylphenyl, bevorzugt $C_{7-40}$-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl, insbesondere $C_{7-20}$-Alkylphenyl,

$R^6$, $R^7$, $R^8$, $R^9$:

- Methyl oder Ethyl, bevorzugt Methyl,

X:

- $CH_2$, $CHR^5$, Sauerstoff (O), Schwefel (S) oder $NR^5$, bevorzugt $CH_2$ und O

Y:

- $N(R^{10})_2$, bevorzugt $NH_2$ und $N(CH_3)_2$,
- Hydroxy (OH),
- $C_{2-20}$-Alkylaminoalkyl, bevorzugt $C_{2-16}$-Alkylaminoalkyl, wie Methylaminomethyl, 2-Methylaminoethyl, Ethylaminomethyl, 2-Ethylaminoethyl und 2-(iso-Propylamino)ethyl,
- $C_{3-20}$-Dialkylaminoalkyl, bevorzugt $C_{3-16}$-Dialkylaminoalkyl, wie Dimethylaminomethyl, 2-Dimethylaminoethyl, 2-Diethylaminoethyl, 2-(Di-n-propylamino)ethyl und 2-(Di-iso-propylamino)ethyl,

j, l:

- eine ganze Zahl von 1 bis 4 (1, 2, 3 oder 4), bevorzugt 2 und 3, besonders bevorzugt 2,

k, m, q:

- eine ganze Zahl von 1 bis 4 (1, 2, 3 oder 4), bevorzugt 2, 3 und 4, besonders bevorzugt 2 und 3,

n:

- eine ganze Zahl von 1 bis 30, bevorzugt eine ganze Zahl von 1 bis 8 (1, 2, 3, 4, 5, 6, 7 oder 8), besonders bevorzugt eine ganze Zahl von 1 bis 6.

Als im erfindungsgemäßen Verfahren einsetzbare Ketone eignen sich unter den o.g. Voraussetzungen praktisch alle aliphatischen und aromatischen Ketone. Die aliphatischen Ketone können geradkettig, verzweigt oder zyklisch sein, die Ketone können Heteroatome enthalten. Die Ketone können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Ketone aminiert werden, so hat man es über die Steuerung der Reaktionsbedingungen in der Hand, Aminoketone, Aminoalkohole, cyclische Amine oder mehrfach aminierte Produkte zu erhalten.

Bevorzugt werden beispielsweise die folgenden Ketone aminierend hydriert:

Aceton, Ethylmethylketon, Methylvinylketon, Isobutylmethylketon, Butanon, 3-Methylbutan-2-on, Diethylketon, Tetralon, Acetophenon, Propiophenon, p-Methylacetophenon, p-Methoxy-acetophenon, m-Methoxy-acetophenon, 1-Acetyl-naphthalin, 2-Acetyl-naphthalin, 1-Phenyl-3-butanon, Cyclobutanon, Cyclopentanon, Cyclopentenon, Cyclohexanon, Cyclohexenon, 2,6-Dimethylcyclohexanon, Cycloheptanon, Cyclododecanon, Acetylaceton, Methylglyoxal und Benzophenon.

Als im erfindungsgemäßen Verfahren einsetzbare Aldehyde eignen sich unter den o.g. Voraussetzungen praktisch alle aliphatischen und aromatischen Aldehyde. Die aliphatischen Aldehyde können geradkettig, verzweigt oder zyklisch sein, die Aldehyde können Heteroatome enthalten. Die Aldehyde können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Aldehyde oder Ketoaldehyde aminiert werden, so hat man es über die Steuerung der Reaktionsbedingungen in der Hand, Aminoalkohole, cyclische Amine oder mehrfach aminierte Produkte zu erhalten.

Bevorzugt werden beispielsweise die folgenden Aldehyde aminierend hydriert:

Formaldehyd, Acetaldehyd, Propionaldehyd, n-Butyraldehyd, Isobutyraldehyd, Pivalinaldehyd, n-Pentanal, n-Hexanal, 2-Ethylhexanal, 2-Methylpentanal, 3-Methylpentanal, 4-Methylpentanal, Glyoxal, Benzaldehyd, p-Methoxybenzaldehyd, p-Methylbenzaldehyd, Phenylacetaldehyd, (p-Methoxy-phenyl)acetaldehyd, (3,4-Dimethoxyphenyl)-acetaldehyd, 4-Formyltetrahydropyran, 3- Formyltetrahydrofuran, 5-Formylvaleronitril, Citronellal, Lysmeral, Acrolein, Methacrolein, Ethylacrolein, Citral, Crotonaldehyd, 3-Methoxypropionaldehyd, 3-Aminopropionaldehyd, Hydroxypivalinaldehyd, Dimethylolpropionaldehyd, Dimethylolbutyraldehyd, Furfural, Glyoxal, Glutaraldehyd sowie hydroformylierte Oligomere und Polymere, wie z. B. hydroformyliertes Polyisobuten (Polyisobutenaldehyd) oder durch Metathese von 1-Penten und Cyclopenten erhaltenes und hydroformyliertes Oligomer.

Als Aminierungsmittel bei der hydrierenden Aminierung von Aldehyden und/oder Ketonen in Gegenwart von Wasserstoff können primäre oder sekundäre, aliphatische oder cycloaliphatische oder aromatische Amine eingesetzt werden.

Aus Di- oder Oligoaldehyden bzw. Di- oder Oligoketonen bzw. Ketoaldehyden lassen sich durch intramolekulare hydrierende Aminierung zyklische Amine, wie z.B. Pyrrolidine, Piperidine, Hexamethylenimine, Piperazine und Morpholine, herstellen.

Bevorzugt werden die primären oder sekundären Amine als Aminierungsmittel zur Herstellung unsymmetrisch substituierter Di- oder Trialkylamine, wie Ethyldiisopropylamin und Ethyldicyclohexylamin verwendet.

Beispielsweise werden die folgenden Mono- und Dialkylamine als Aminierungsmittel verwendet: Methylamin, Dimethylamin, Ethylamin, Diethylamin, n-Propylamin, Di-n-propyl-amin, iso-Propylamin, Di-isopropyl-amin, Dimethylmorpholin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Bis(2-ethylhexyl)amin, Cyclohexylamin, Anilin, Toluidin, Piperidin, Morpholin und Pyrrolidin.

Mit dem erfindungsgemäßen Verfahren besonders bevorzugt hergestellte Amine sind zum Beispiel N,N-Di ($C_{1-4}$-alkyl)cyclohexylamin (aus Cyclohexanon und Di($C_{1-4}$-alkyl)-amin), Dicyclohexylamin (aus Cyclohexanon und Cyclohexylamin), N,N-Dimethyl-N-propylamin (aus n-Propanal und Dimethylamin), N,N-Dimethyl-N-isopropylamin (aus Aceton und DMA), N-Ethyl-N,N-diisopropylamin (aus Acetaldehyd und N,N-Diisopropylamin), Tris(2-ethylhexyl)amin (aus 2-Ethylhexanal und Bis(2-ethylhexyl)amin), und cis-4-[3-(4-tert-Butylphenyl)-2-methylpropyl]-2,6-dimethylmorpholin (aus Lysmeral und cis-2,6-Dimethylmorpholin).

Ganz besonders bevorzugte erfindungsgemäße Verfahren sind diejenigen

a) zur Herstellung von N-Ethyl-N,N-diisopropylamin (Hünig-Base) durch Umsetzung von Acetaldehyd mit Diisopropylamin, bevorzugt unter Einsatz eines Pd/C-Suspensionskatalysators, wobei die Umsetzung bevorzugt bei einem Absolutdruck im Bereich von 20 bis 100 bar, besonders 25 bis 80 bar, weiter besonders 40 bis 75 bar, und bevorzugt bei einer Temperatur im Bereich von 70 bis 170 °C, besonders 80 bis 140 °C, weiter besonders 90 bis 120 °C, durchgeführt wird, und

b) zur Herstellung von Tris(2-ethylhexyl)amin durch Umsetzung von 2-Ethylhexanal mit Bis(2-ethylhexyl)amin, bevorzugt unter Einsatz eines Pd/C-Suspensionskatalysators, wobei die Umsetzung bevorzugt bei einem Absolutdruck im Bereich von 20 bis 100 bar, besonders 25 bis 80 bar, weiter besonders 40 bis 75 bar, und bevorzugt bei einer Temperatur im Bereich von 100 bis 170 °C, besonders 120 bis 160 °C, durchgeführt wird.

Alle ppm-Angaben in diesem Dokument beziehen sich auf das Gewicht.

## Beispiele

Laborversuche zur Synthese von N-Ethyl-N,N-diisopropylamin (EDIPA)

Die Laborversuche wurden in einem 500 ml Rührautoklaven der Firma Büchi durchgeführt. Diisopropylamin (DIPA) wurde im Autoklaven vorgelegt, in dem der jeweilige Suspensionskatalysator eingebaut war. Der Reaktor wurde mit Stickstoff gespült. Ein geringer Wasserstoffdruck wurde aufgepresst, und die Mischung wurde auf Reaktionstemperatur erhitzt. Der Wasserstoffdruck wurde nach Erreichen der Reaktionstemperatur auf den gewünschten Reaktionsdruck erhöht. Der Acetaldehyd wurde im Versuchsverlauf innerhalb von 1 bis 5 Stunden mit einer Pumpe bei Reaktionsdruck zugegeben (semibatch-Fahrweise). Regelmäßig wurden Proben aus dem Reaktor entnommen und per Gaschromatographie analysiert. Die zugegebene Aldehydmenge wurde so bemessen, dass ein DIPA-Umsatz von mindestens 95 % erreicht wurde, insgesamt resultierte ein molarer Acetaldehyd-Überschuss um 20 %. Die Rührerdrehzahl betrug 300 bis 1200 U/min. Die Reaktionstemperatur betrug 100 °C, der Reaktionsdruck 25 bar. Als Katalysatoren wurden kohlegeträgerte, palladiumhaltige Suspensionskatalysatoren mit 5 % Pd auf Kohle (Wassergehalt ca. 50 %) eingesetzt. Eine spezifische Katalysatormenge von 0,03 g (Katalysator, ber. 100 %)/g (Diisopropylamin) wurde verwendet, also 6 Gew.-% wasserfeuchter Katalysator, bezogen auf DIPA.

Die Reaktionsausträge wurden mittels Gaschromatographie analysiert.

Als Messprogramm wurde eingesetzt: Trennsäule DB1, Länge 60 m; Innendurchmesser 0,32 mm; Trägergas Helium; Temperaturprogramm: 80 °C, danach mit 5 °C/Minute auf 260 °C, abschließend 10 Minuten isotherm bei 260 °C.

In der Tabelle 1 sind die Ergebnisse der verschiedenen Versuche gezeigt (Zusammensetzung der organischen

Phase in GC-Flächenprozent). Eine mehrfache Katalysatorrückführung gelang ohne merklichen Rückgang der Katalyseaktivität (Einträge 4 bis 6 in Tabelle 1).

Tabelle 1: Laborversuche zur EDIPA-Synthese

| Nr. | Zugabedauer h | Reaktionszeit h | Ethanol % | DIPA % | EDIPA % | Diisopropylbutylamin % | Diisopropylbutanolamin % | Sonstige % |
|---|---|---|---|---|---|---|---|---|
| 1 | 1 | 4 | 1,7 | 0,4 | 94,9 | 1,1 | 1,1 | 1,9 |
| 2 | 3 | 6 | 1,2 | 1,1 | 94,6 | 1,3 | 1,0 | 1,8 |
| 3 | 5 | 8 | 1,2 | 0,7 | 93,4 | 1,8 | 1,5 | 2,9 |
| 4 | 3 | 6 | 1,4 | 1,5 | 95,2 | 0,9 | 0,5 | 1,0 |
| 5* | 3 | 6 | 0,9 | 0,1 | 95,1 | 1,5 | 0,8 | 2,4 |
| 6* | 3 | 6 | 0,7 | 0,1 | 95,0 | 1,7 | 0,9 | 2,6 |
| *) Katalysatorrückführung aus Versuch Nr. 4 | | | | | | | | |

Technische Synthese von N-Ethyl-N,N-diisopropylamin

Der Versuch wurde in einem Rührautoklav durchgeführt. 2170 bis 4100 kg Diisopropylamin (DIPA) wurden im Reaktor vorgelegt, in dem 140 kg des kohlegeträgerten, palladiumhaltigen Suspensionskatalysators mit 5 % Pd auf Kohle (Wassergehalt ca. 50 %) eingebaut war. Der Reaktor wurde mit Stickstoff gespült. Ein geringer Wasserstoffdruck wurde eingestellt und anschließend wurde auf Reaktionstemperatur erhitzt. Sobald die Reaktionstemperatur erreicht war, wurde der Wasserstoffdruck bis auf den gewünschten Reaktionsdruck erhöht. Der Acetaldehyd (1130 bis 2233 kg) wurde im Versuchsverlauf mit einer Dosiergeschwindigkeit von 120 bis 300 kg/h zugegeben. Im Versuchsverlauf wurde der DIPA-Umsatz ermittelt und die Aldehydmenge so bemessen, dass ein Umsatz von mindestens 95 % erreicht wurde. Es ergaben sich daraus molare Aldehyd-Überschüsse von 20 bis 64 %.

In der Tabelle 2 sind die Ergebnisse der 13 Reaktionspartien gezeigt (Reaktionsparameter und Zusammensetzung der organischen Phase in GC-Flächenprozent). Eine 12-fache Katalysatorrückführung gelang ohne merklichen Rückgang der Katalyseaktivität.

Tabelle 2: Betriebsversuch zur EDIPA-Synthese, Reaktionsparameter und Zusammensetzung der organischen Phase des Reaktionsaustrages

| Nr. | Temperatur °C | Druck bar | Ethanol % | DIPA % | EDIPA % | Diisopropylbutylamin % | Diisopropylbutanolamin % | Sonstige % |
|---|---|---|---|---|---|---|---|---|
| 1 | 100 | 25 | 8,0 | 2,1 | 81,8 | 3,2 | 0,9 | 4,0 |
| 2 | 100 | 25 | 1,3 | 2,8 | 85,3 | 6,3 | 1,3 | 3,0 |
| 3 | 100 | 50 | 0,8 | 1,9 | 86,0 | 6,7 | 1,1 | 3,5 |
| 4 | 100 | 25 | 0,7 | 1,2 | 81,6 | 11,0 | 1,5 | 4,1 |
| 5 | 120 | 25 | 0,7 | 1,7 | 86,9 | 7,3 | 0,7 | 2,8 |
| 6 | 100 | 25 | 0,5 | 2,6 | 83,4 | 9,6 | 1,0 | 2,9 |
| 7 | 120 | 50 | 0,9 | 1,9 | 88,1 | 6,3 | 0,6 | 2,2 |
| 8 | 120 | 50 | 1,4 | 2,0 | 85,2 | 7,6 | 0,5 | 3,4 |
| 9 | 120 | 50 | 1,2 | 2,9 | 86,4 | 6,7 | 0,5 | 2,4 |
| 10 | 120 | 50 | 1,4 | 2,5 | 82,3 | 9,8 | 0,5 | 3,6 |
| 11 | 130 | 50 | 1,7 | 2,9 | 86,5 | 6,3 | 0,3 | 2,5 |
| 12 | 130 | 50 | 1,8 | 2,0 | 84,7 | 7,9 | 0,3 | 3,4 |
| 13 | 130 | 50 | 1,6 | 3,0 | 84,6 | 7,9 | 0,3 | 2,5 |

Technische Synthese von Tris(2-ethylhexyl)amin (Tris-EH)

Der Versuch wurde in einem Rührautoklav durchgeführt. 4000 kg Bis(2-ethylhexylamin (Di-EH) wurde im Reaktor vorgelegt, in dem 40 bis 150 kg des kohlegeträgerten, palladiumhaltigen Suspensionskatalysators mit 5 % Pd auf Kohle (Wassergehalt ca. 50 %) eingebaut war. Der Reaktor wurde mit Stickstoff gespült. Ein geringer Wasserstoffdruck wurde eingestellt und anschließend wurde auf Reaktionstemperatur erhitzt. Sobald die Reaktionstemperatur erreicht war, wurde der Wasserstoffdruck bis auf den gewünschten Reaktionsdruck erhöht. Der Aldehyd 2-Ethylhexanal (2-EH, 2120 bis 2320 kg) wurde nachfolgend kontinuierlich zugegeben. Im Versuchsverlauf wurde der Di-EH-Umsatz ermittelt und die Aldehydmenge so bemessen, dass ein Umsatz von mindestens 95 % erreicht wurde. Es ergaben sich daraus molare Aldehyd-Überschüsse von 0,1 bis 9 %. Als überwiegendes Nebenprodukt wurden geringe Mengen an Ethylhexanol (E-ol) gebildet.

In der Tabelle 3 sind die Ergebnisse der 33 Reaktionspartien gezeigt (Reaktionsparameter und Zusammensetzung der organischen Phase in GC-Flächenprozent). Nach der 1., 3., 8. und 15. Partie wurde frischer Katalysator in den Reaktor gegeben, wodurch sich die Katalysatormenge auf schließlich 150 kg erhöhte. Ab der Partie 16 wurde eine 17-fache Katalysatorrückführung durchgeführt, ohne merklichen Rückgang der Katalyseaktivität.

Tabelle 3: Betriebsversuch Tris(2-ethylhexyl)amin, Reaktionsparameter und Zusammensetzung der organischen Phase des Reaktionsaustrages

| Nr. | Temperatur °C | Druck bar | Reaktionszeit h | Tris-EH % | Di-EH % | 2-EH % | E-ol % | Katalysatormenge kg | Zugabe 2-EH kg |
|---|---|---|---|---|---|---|---|---|---|
| 1 | 80 | 50 | 52 | 86,2 | 2,9 | 0,8 | 8,7 | 40 | 2120 |
| 2 | 123 | 50 | 35 | 92,1 | 1,7 | 0,2 | 4,9 | 60 | 2320 |
| 3 | 127 | 50 | 31 | 92,8 | 2,0 | 0,4 | 5,9 | 60 | 2320 |
| 4 | 127 | 50 | 17 | 94,1 | 1,3 | 0,5 | 4,4 | 80 | 2120 |
| 5 | 135 | 50 | 23 | 93,7 | 0,5 | 3,9 | 0,6 | 80 | 2320 |
| 6 | 135 | 60 | 13 | 88,6 | 3,0 | 3,5 | 3,6 | 80 | 2120 |
| 7 | 135 | 60 | 19 | 89,6 | 3,5 | 0,8 | 4,9 | 80 | 2120 |
| 8 | 135 | 60 | 12 | 91,0 | 3,3 | 1,5 | 2,5 | 100 | 2120 |
| 9 | 135 | 60 | 10 | 91,4 | 2,3 | 1,9 | 3,5 | 100 | 2326 |
| 10 | 135 | 40 | 12 | 93,7 | 1,0 | 1,6 | 2,8 | 100 | 2320 |
| 11 | 135 | 40 | 13 | 93,5 | 2,3 | 1,3 | 1,8 | 100 | 2222 |
| 12 | 135 | 40 | 10 | 92,2 | 3,2 | 2,0 | 1,6 | 100 | 2222 |
| 13 | 135 | 40 | 11 | 92,3 | 3,1 | 1,8 | 1,8 | 100 | 2222 |
| 14 | 135 | 40 | 9 | 91,7 | 3,3 | 2,4 | 1,6 | 100 | 2222 |
| 15 | 135 | 40 | 5 | 93,3 | 1,5 | 2,5 | 1,6 | 150 | 2307 |
| 16 | 135 | 40 | 5 | 93,6 | 2,0 | 2,0 | 1,3 | 150 | 2228 |
| 17 | 135 | 30 | 6 | 94,3 | 1,7 | 1,8 | 1,1 | 150 | 2225 |
| 18 | 135 | 20 | 9 | 95,7 | 1,4 | 2,2 | 0,9 | 150 | 2223 |
| 19 | 140 | 20 | 6 | 91,4 | 3,7 | 2,6 | 0,8 | 150 | 2224 |
| 20 | 140 | 40 | 5 | 92,7 | 2,6 | 2,4 | 1,2 | 150 | 2221 |
| 21 | 140 | 40 | 7 | 94,4 | 1,0 | 1,1 | 2,2 | 150 | 2253 |
| 22 | 140 | 40 | 5 | 89,6 | 4,6 | 2,2 | 2,5 | 150 | 2220 |
| 23 | 140 | 40 | 7 | 94,5 | 1,9 | 1,6 | 1,1 | 150 | 2224 |
| 24 | 140 | 40 | 6 | 93,5 | 2,1 | 2,3 | 1,1 | 150 | 2238 |
| 25 | 140 | 40 | 5 | 93,2 | 2,1 | 1,3 | 1,3 | 150 | 2222 |

(fortgesetzt)

| Nr. | Temperatur °C | Druck bar | Reaktionszeit h | Tris-EH % | Di-EH % | 2-EH % | E-ol % | Katalysatormenge kg | Zugabe 2-EH kg |
|---|---|---|---|---|---|---|---|---|---|
| 26 | 140 | 40 | 6 | 94,2 | 2,2 | 1,2 | 1,1 | 150 | 2233 |
| 27 | 140 | 40 | 6 | 92,6 | 3,2 | 1,7 | 1,0 | 150 | 2223 |
| 28 | 140 | 40 | 14 | 96,0 | 0,2 | 0,4 | 2,0 | 150 | 2226 |
| 29 | 150 | 60 | 13 | 95,6 | 0,8 | 0,0 | 2,7 | 150 | 2236 |
| 30 | 150 | 60 | 9 | 94,5 | 2,7 | 0,0 | 1,6 | 150 | 2222 |
| 31 | 150 | 60 | 11 | 93,0 | 2,0 | 0,0 | 3,6 | 150 | 2222 |
| 32 | 155 | 60 | 14 | 95,8 | 0,8 | 0,1 | 2,3 | 150 | 2222 |
| 33 | 155 | 60 | 13 | 95,4 | 1,4 | 0,1 | 1,8 | 150 | 2236 |

**Patentansprüche**

1. Verfahren zur Herstellung eines Amins durch Umsetzung eines Aldehyds und/oder Ketons mit Wasserstoff und einer Stickstoffverbindung, ausgewählt aus der Gruppe primärer und sekundärer Amine, in Gegenwart eines Heterogenkatalysators, **dadurch gekennzeichnet, dass** die Umsetzung
unter Einsatz eines Suspensionskatalysators als Heterogenkatalysator erfolgt und
in semibatch-Fahrweise durchgeführt wird, bei der die Stickstoffverbindung als ein Reaktionspartner im Reaktionsgefäß vorgelegt und der Aldehyd und/oder das Keton als der andere Reaktionspartner im Reaktionsverlauf zugegeben wird, und im Reaktionsverlauf in Abhängigkeit vom erreichten Umsatz der Stickstoffverbindung der Aldehyd und/oder das Keton portionsweise oder kontinuierlich zum Reaktionsgemisch gegeben wird, bis ein Umsatz der Stickstoffverbindung von mindestens 95 % resultiert,
und der Katalysator ganz oder teilweise nach der Reaktionspartie für die nächste Reaktionspartie zum erneuten Einsatz im Reaktionsgefäß verbleibt und wieder eingesetzt wird, wobei unter "teitweisem Verbleib des Katalysators im Reaktionsgefäß" zu verstehen ist, dass mindestens 30 Gew.% des ursprünglich eingesetzten Katalysators nach Durchführung der Umsetzung und Abtrennung der organischen Reaktionsprodukte im Reaktionsgefäß verbleiben.

2. Verfahren nach Anspruch 1, **dadurch gekennzeichnet, dass** der Katalysator ganz oder teilweise nach der Reaktionspartie zusammen mit gebildeter wässriger Phase oder einem Teil von gebildeter wässriger Phase, zum erneuten Einsatz im Reaktionsgefäß verbleibt und wieder eingesetzt wird.

3. Verfahren nach Anspruch 1 oder 2, **dadurch gekennzeichnet, dass** der Katalysator ganz oder teilweise nach der Reaktionspartie zum erneuten Einsatz im Reaktionsgefäß verbleibt und ohne eine Spülung oder Regenerierung wieder eingesetzt wird.

4. Verfahren nach einem der vorhergehenden Ansprüche, **dadurch gekennzeichnet, dass** im Reaktionsverlauf in Abhängigkeit vom erreichten Umsatz der Stickstoffverbindung der Aldehyd und/oder das Keton portionsweise oder kontinuierlich zum Reaktionsgemisch gegeben wird, bis ein Umsatz der Stickstoffverbindung von mindestens 96 % resultiert.

5. Verfahren nach einem der vorhergehenden Ansprüche, **dadurch gekennzeichnet, dass** die Umsetzung bei einer Temperatur im Bereich von 15 bis 180 °C durchgeführt wird.

6. Verfahren nach einem der vorhergehenden Ansprüche, **dadurch gekennzeichnet, dass** in der Umsetzung die Stickstoffverbindung und der Katalysator vorgelegt werden und der Aldehyd und/oder das Keton mit einer Geschwindigkeit zugegeben wird, die so gewählt ist, dass die gewünschte Maximaltemperatur der Reaktion nicht überschritten wird.

7. Verfahren nach einem der vorhergehenden Ansprüche, **dadurch gekennzeichnet, dass** das Anfahren der Reaktion in folgender Reihenfolge erfolgt:

(a) der Reaktor wird mit der Stickstoffverbindung und Katalysator beschickt,
(b) der Reaktor wird mit Stickstoff gespült,
(c) ein Wasserstoffdruck wird eingestellt, der geringer als der spätere Reaktionsdruck ist,
(d) der Reaktor wird auf Reaktionstemperatur aufgeheizt,
(e) der Wasserstoffdruck wird auf Reaktionsdruck erhöht,
(f) der Aldehyd und/oder das Keton wird portionsweise oder kontinuierlich zum Reaktionsgemisch gegeben.

**8.** Verfahren nach einem der vorhergehenden Ansprüche, **dadurch gekennzeichnet, dass** die Umsetzung in der Flüssigphase durchgeführt wird.

**9.** Verfahren nach einem der Ansprüche 1 bis 7, **dadurch gekennzeichnet, dass** die Umsetzung in der Flüssigphase oder in einer gemischten Flüssig-/Gasphase mit mindestens 50 Gew.-% des Reaktionsgemisches in der Flüssigphase durchgeführt wird.

**10.** Verfahren nach einem der vorhergehenden Ansprüche, **dadurch gekennzeichnet, dass** die Umsetzung bei einem Absolutdruck (= Reaktionsdruck) im Bereich von 1 bis 120 bar durchgeführt wird.

**11.** Verfahren nach einem der vorhergehenden Ansprüche, **dadurch gekennzeichnet, dass** der Aldehyd und/oder das Keton in der 1,0- bis 3,5-fachen molaren Menge bezogen auf die Stickstoffverbindung eingesetzt wird.

**12.** Verfahren nach einem der vorhergehenden Ansprüche, **dadurch gekennzeichnet, dass** die katalytisch aktiven Metalle oder die Metalle in deren Verbindungen im Suspensionskatalysator ausgewählt sind aus den Elementen der Gruppen 8 und/oder 9 und/oder 10 und/oder 11 des Periodensystems der Elemente (IUPAC notation 1985).

**13.** Verfahren nach einem der vorhergehenden Ansprüche, **dadurch gekennzeichnet, dass** es sich bei dem Suspensionskatalysator um einen geträgerten Übergangsmetallkatalysator handelt, der als katalytisch aktives Metall Pd enthält.

**14.** Verfahren nach dem vorhergehenden Anspruch, **dadurch gekennzeichnet, dass** der geträgerte Übergangsmetallkatalysator Aktivkohle als Träger aufweist.

**15.** Verfahren nach dem vorvorhergehenden Anspruch, **dadurch gekennzeichnet, dass** der geträgerte Übergangsmetallkatalysator Aluminiumoxid als Träger aufweist.

**16.** Verfahren nach einem der vorhergehenden Ansprüche, **dadurch gekennzeichnet, dass** der Suspensionskatalysator einen Edelmetallgehalt im Bereich von 0,1 bis 25 Gew.% (bezogen auf die Gesamtmasse des Katalysators, ohne Wasser) aufweist.

**17.** Verfahren nach einem der Ansprüche 1 bis 15, **dadurch gekennzeichnet, dass** der Suspensionskatalysator einen Edelmetallgehalt im Bereich von 0,5 bis 15 Gew.% (bezogen auf die Gesamtmasse des Katalysators, ohne Wasser) aufweist.

**18.** Verfahren nach einem der vorhergehenden Ansprüche, **dadurch gekennzeichnet, dass** der Suspensionskatalysator einen Wassergehalt im Bereich von 1 bis 70 Gew.% aufweist.

**19.** Verfahren nach einem der vorhergehenden Ansprüche, **dadurch gekennzeichnet, dass** man den Katalysator in einer solchen Menge einsetzt, dass die Menge an Katalysator (wasserfrei gerechnet), bezogen auf die Menge an umzusetzendem primären oder sekundären Amin, im Bereich von 0,1 bis 20,0 Gew.-% beträgt.

**20.** Verfahren nach einem der vorhergehenden Ansprüche, **dadurch gekennzeichnet, dass** man die Umsetzung ohne Zusatz eines Promotors oder Hilfsstoffes durchführt.

**21.** Verfahren nach einem der Ansprüche 1 bis 20 zur Herstellung von N-Ethyl-N,N-diisopropylamin (Hünig-Base) durch Umsetzung von Acetaldehyd mit Diisopropylamin.

**22.** Verfahren nach dem vorhergehenden Anspruch, **dadurch gekennzeichnet, dass** die Umsetzung unter Einsatz eines Pd/C-Suspensionskatalysators durchgeführt wird.

**23.** Verfahren nach einem der beiden vorhergehenden Ansprüche, **dadurch gekennzeichnet, dass** die Umsetzung bei einem Absolutdruck im Bereich von 20 bis 100 bar und einer Temperatur im Bereich von 70 bis 170 °C durchgeführt wird.

**24.** Verfahren nach einem der Ansprüche 1 bis 20 zur Herstellung von Tris(2-ethylhexyl)amin durch Umsetzung von 2-Ethylhexanal mit Bis(2-ethylhexyl)amin.

**25.** Verfahren nach dem vorhergehenden Anspruch, **dadurch gekennzeichnet, dass** die Umsetzung unter Einsatz eines Pd/C-Suspensionskatalysators durchgeführt wird.

**26.** Verfahren nach einem der beiden vorhergehenden Ansprüche, **dadurch gekennzeichnet, dass** die Umsetzung bei einem Absolutdruck im Bereich von 20 bis 100 bar und einer Temperatur im Bereich von 100 bis 170 °C durchgeführt wird.

**27.** Verfahren nach einem der Ansprüche 1 bis 20 zur Herstellung von cis-4-[3-(4-tert-Butylphenyl)-2-methylpropyl]-2,6-dimethylmorpholin durch Umsetzung von Lysmeral mit cis-2,6-Dimethylmorpholin.

**28.** Verfahren nach einem der Ansprüche 1 bis 20 zur Herstellung von N,N-Dimethylcyclohexylamin durch Umsetzung von Cyclohexanon mit Dimethylamin.

**29.** Verfahren nach einem der Ansprüche 1 bis 20 zur Herstellung von N,N-Dimethyl-N-isopropylamin durch Umsetzung von Aceton mit Dimethylamin.

**30.** Verfahren nach einem der Ansprüche 1 bis 20 zur Herstellung von Dicyclohexylamin durch Umsetzung von Cyclohexanon mit Cyclohexylamin.

**31.** Verfahren nach einem der Ansprüche 1 bis 20 zur Herstellung von N,N-Dimethyl-N-n-propylamin durch Umsetzung von Propanal mit Dimethylamin.

**Claims**

**1.** A process for preparing an amine by reacting an aldehyde and/or ketone with hydrogen and a nitrogen compound selected from the group consisting of primary and secondary amines in the presence of a heterogeneous catalyst, wherein the reaction
is carried out using a suspended catalyst as heterogeneous catalyst and
is carried out in the semibatch mode in which the nitrogen compound as one reactant is placed in the reaction vessel and the aldehyde and/or the ketone as the other reactant is added during the course of the reaction and the aldehyde and/or the ketone is added in portions or continuously to the reaction mixture during the course of the reaction as a function of the achieved conversion of the nitrogen compound until a conversion of the nitrogen compound of at least 95% results,
and all or part of the catalyst remains in the reaction vessel after the reaction batch and is reused for the next reaction batch, "part of the catalyst remaining in the reaction vessel" meaning that at least 30% by weight of the catalyst originally used remains in the reaction vessel after the reaction has been carried out and the organic reaction products have been separated off.

**2.** The process according to claim 1, wherein all or part of the catalyst remains in the reaction vessel together with the aqueous phase formed or part of the aqueous phase formed after the reaction batch and is reused.

**3.** The process according to claim 1 or 2, wherein all or part of the catalyst remains in the reaction vessel after the reaction batch and is reused without rinsing or regeneration.

**4.** The process according to any of the preceding claims, wherein the aldehyde and/or the ketone is added in portions or continuously to the reaction mixture during the course of the reaction as a function of the achieved conversion of the nitrogen compound until a conversion of the nitrogen compound of at least 96% results.

**5.** The process according to any of the preceding claims, wherein the reaction is carried out at a temperature in the range from 15 to 180°C.

**6.** The process according to any of the preceding claims, wherein, in the reaction, the nitrogen compound and the catalyst are initially charged and the aldehyde and/or the ketone is/are added at a rate which is selected so that the desired maximum temperature of the reaction is not exceeded.

**7.** The process according to any of the preceding claims, wherein the reaction is started up in the following order:

(a) the reactor is charged with the nitrogen compound and catalyst,
(b) the reactor is flushed with nitrogen,
(c) a hydrogen pressure which is lower than the later reaction pressure is set,
(d) the reactor is heated to the reaction temperature,
(e) the hydrogen pressure is increased to the reaction pressure,
(f) the aldehyde and/or the ketone is added in portions or continuously to the reaction mixture.

**8.** The process according to any of the preceding claims, wherein the reaction is carried out in the liquid phase.

**9.** The process according to any of claims 1 to 7, wherein the reaction is carried out in the liquid phase or in a mixed liquid/gas phase with at least 50% by weight of the reaction mixture in the liquid phase.

**10.** The process according to any of the preceding claims, wherein the reaction is carried out at an absolute pressure (= reaction pressure) in the range from 1 to 120 bar.

**11.** The process according to any of the preceding claims, wherein the aldehyde and/or the ketone is/are used in 1.0- to 3.5-fold molar amount based on the nitrogen compound.

**12.** The process according to any of the preceding claims, wherein the catalytically active metals or the metals in their compounds in the suspended catalyst are selected from among the elements of groups 8 and/or 9 and/or 10 and/or 11 of the Periodic Table of the Elements (IUPAC notation 1985).

**13.** The process according to any of the preceding claims, wherein the suspended catalyst is a supported transition metal catalyst comprising Pd as catalytically active metal.

**14.** The process according to the preceding claim, wherein the supported transition metal catalyst comprises activated carbon as support.

**15.** The process according to the preceding claim, wherein the supported transition metal catalyst comprises aluminum oxide as support.

**16.** The process according to any of the preceding claims, wherein the suspended catalyst has a noble metal content in the range from 0.1 to 25% by weight (based on the total mass of the catalyst, without water).

**17.** The process according to any of claims 1 to 15, wherein the suspended catalyst has a noble metal content in the range from 0.5 to 15% by weight (based on the total mass of the catalyst, without water).

**18.** The process according to any of the preceding claims, wherein the suspended catalyst has a water content in the range from 1 to 70% by weight.

**19.** The process according to any of the preceding claims, wherein the catalyst is used in such an amount that the ratio of the amount of catalyst (calculated on a water-free basis) to the amount of primary or secondary amine to be reacted is in the range from 0.1 to 20.0% by weight.

**20.** The process according to any of the preceding claims, wherein the reaction is carried out without addition of a promoter or auxiliary.

**21.** The process according to any of claims 1 to 20 for preparing N-ethyl-N,N-diisopropylamine (HOnig base) by reacting acetaldehyde with diisopropylamine.

**22.** The process according to the preceding claim, wherein the reaction is carried out using a suspended Pd/C catalyst.

23. The process according to either of the two preceding claims, wherein the reaction is carried out at an absolute pressure in the range from 20 to 100 bar and a temperature in the range from 70 to 170°C.

24. The process according to any of claims 1 to 20 for preparing tris(2-ethylhexyl)-amine by reacting 2-ethylhexanal with bis(2-ethylhexyl)amine.

25. The process according to the preceding claim, wherein the reaction is carried out using a suspended Pd/C catalyst.

26. The process according to either of the two preceding claims, wherein the reaction is carried out at an absolute pressure in the range from 20 to 100 bar and a temperature in the range from 100 to 170°C.

27. The process according to any of claims 1 to 20 for preparing cis-4-[3-(4-tert-butylphenyl)-2-methylpropyl]-2,6-dimethylmorpholine by reacting lysmeral with cis-2,6-dimethylmorpholine.

28. The process according to any of claims 1 to 20 for preparing N,N-dimethyl-cyclohexylamine by reacting cyclohexanone with dimethylamine.

29. The process according to any of claims 1 to 20 for preparing N,N-dimethyl-N-isopropylamine by reacting acetone with dimethylamine.

30. The process according to any of claims 1 to 20 for preparing dicyclohexylamine by reacting cyclohexanone with cyclohexylamine.

31. The process according to any of claims 1 to 20 for preparing N,N-dimethyl-N-n-propylamine by reacting propanal with dimethylamine.

**Revendications**

1. Procédé pour la préparation d'une amine par transformation d'un aldéhyde et/ou d'une cétone avec de l'hydrogène et un composé azoté, choisi dans le groupe formé par les amines primaires et secondaires, en présence d'un catalyseur hétérogène, **caractérisé en ce que** la transformation
a lieu en utilisant un catalyseur en suspension en tant que catalyseur hétérogène et
est réalisée dans un mode semi-continu, dans lequel le composé azoté est disposé au préalable dans le récipient de réaction en tant que partenaire de réaction et l'aldéhyde et/ou la cétone est ajouté(e) comme l'autre partenaire de réaction au cours de la réaction et on ajoute, au cours de la réaction, en fonction de la conversion atteinte du composé azoté, l'aldéhyde et/ou la cétone par portions ou en continu au mélange réactionnel, jusqu'à ce qu'on atteigne une conversion du composé azoté d'au moins 95%,
et le catalyseur reste totalement ou partiellement après le lot de réaction pour le lot de réaction suivant, pour être réutilisé, dans le récipient de réaction et est à nouveau utilisé, en entendant par "le catalyseur reste partiellement dans le récipient de réaction" qu'au moins 30% en poids du catalyseur utilisé au départ restent dans le récipient de réaction après la réalisation de la transformation et la séparation des produits de réaction organiques.

2. Procédé selon la revendication 1, **caractérisé en ce que** le catalyseur reste totalement ou partiellement après le lot de réaction, ensemble avec la phase aqueuse formée ou une partie de la phase aqueuse formée, pour une nouvelle utilisation, dans le récipient de réaction et est à nouveau utilisé.

3. Procédé selon la revendication 1 ou 2, **caractérisé en ce que** le catalyseur reste totalement ou partiellement après le lot de réaction, pour une nouvelle utilisation, dans le récipient de réaction et est à nouveau utilisé sans rinçage ou régénération.

4. Procédé selon l'une quelconque des revendications précédentes, **caractérisé en ce qu'**au cours de la réaction, on ajoute, en fonction de la conversion atteinte du composé azoté, l'aldéhyde et/ou la cétone par portions ou en continu au mélange réactionnel, jusqu'à ce qu'on atteigne une conversion du composé azoté d'au moins 96%.

5. Procédé selon l'une quelconque des revendications précédentes, **caractérisé en ce que** la transformation est réalisée à une température dans la plage de 15 à 180°C.

6. Procédé selon l'une quelconque des revendications précédentes, **caractérisé en ce qu'**on dispose au préalable, dans la transformation, le composé azoté et le catalyseur et on ajoute l'aldéhyde et/ou la cétone à une vitesse qui est choisie pour que la température ne passe pas au-dessus de la température maximale souhaitée de la réaction.

7. Procédé selon l'une quelconque des revendications précédentes, **caractérisé en ce que** le démarrage de la réaction a lieu dans l'ordre suivant :

(a) le réacteur est alimenté en composé azoté et en catalyseur,
(b) le réacteur est rincé à l'azote,
(c) une pression d'hydrogène est réglée, qui est inférieure à la pression de réaction ultérieure,
(d) le réacteur est chauffé à la température de réaction.
(e) la pression d'hydrogène est augmentée à la pression de réaction,
(f) l'aldéhyde et/ou la cétone est ajouté(e) par portions ou en continu au mélange réactionnel.

8. Procédé selon l'une quelconque des revendications précédentes, **caractérisé en ce que** la transformation est réalisée en phase liquide.

9. Procédé selon l'une quelconque des revendications 1 à 7, caractérisé en ce la transformation est réalisée en phase liquide ou dans une phase liquide/gazeuse mixte contenant au moins 50% en poids du mélange réactionnel dans la phase liquide.

10. Procédé selon l'une quelconque des revendications précédentes, **caractérisé en ce que** la transformation est réalisée à une pression absolue (= pression de réaction) dans la plage de 1 à 120 bars.

11. Procédé selon l'une quelconque des revendications précédentes, **caractérisé en ce que** l'aldéhyde et/ou la cétone est utilisé(e) en une quantité représentant 1,0 à 3,5 fois la quantité molaire par rapport au composé azoté.

12. Procédé selon l'une quelconque des revendications précédentes, **caractérisé en ce que** les métaux catalytiquement actifs ou les métaux dans leurs composés dans le catalyseur en suspension sont choisis parmi les éléments des groupes 8 et/ou 9 et/ou 10 et/ou 11 du système périodique des éléments (notation IUPAC 1985).

13. Procédé selon l'une quelconque des revendications précédentes, **caractérisé en ce qu'**il s'agit, pour le catalyseur en suspension, d'un catalyseur à base d'un métal de transition, supporté, qui contient du Pd comme métal catalytiquement actif.

14. Procédé selon la revendication précédente, **caractérisé en ce que** le catalyseur à base d'un métal de transition, supporté, contient du charbon actif comme support.

15. Procédé selon la revendication précédente, **caractérisé en ce que** le catalyseur à base d'un métal de transition, supporté, contient de l'oxyde d'aluminium comme support.

16. Procédé selon l'une quelconque des revendications précédentes, **caractérisé en ce que** le catalyseur en suspension présente une teneur en métal noble dans la plage de 0,1 à 25% en poids (par rapport à la masse totale du catalyseur, sans eau).

17. Procédé selon l'une quelconque des revendications 1 à 15, **caractérisé en ce que** le catalyseur en suspension présente une teneur en métal noble dans la plage de 0,5 à 15% en poids (par rapport à la masse totale du catalyseur, sans eau).

18. Procédé selon l'une quelconque des revendications précédentes, **caractérisé en ce que** le catalyseur en suspension présente une teneur en eau dans la plage de 1 à 70% en poids.

19. Procédé selon l'une quelconque des revendications précédentes, **caractérisé en ce qu'**on utilise le catalyseur en une quantité telle que la quantité de catalyseur (calculée sous forme anhydre) par rapport à la quantité d'amine primaire ou secondaire à transformer, se situe dans la plage de 0,1 à 20,0% en poids.

20. Procédé selon l'une quelconque des revendications précédentes, **caractérisé en ce qu'**on réalise la transformation sans addition d'un promoteur ou d'un adjuvant.

**21.** Procédé selon l'une quelconque des revendications 1 à 20 pour la préparation de N-éthyl-N,N-diisopropylamine (base de Hünig) par transformation d'acétaldéhyde avec de la diisopropylamine.

**22.** Procédé selon la revendication précédente, **caractérisé en ce que** la transformation est réalisée en utilisant un catalyseur en suspension de type Pd/C.

**23.** Procédé selon l'une quelconque des deux revendications précédentes, **caractérisé en ce que** la transformation est réalisée à une pression absolue dans la plage de 20 à 100 bars et à une température dans la plage de 70 à 170°C.

**24.** Procédé selon l'une quelconque des revendications 1 à 20 pour la préparation de tris(2-éthylhexyl)amine par transformation de 2-éthylhexanal avec de la bis(2-éthylhexyl)amine.

**25.** Procédé selon la revendication précédente, **caractérisé en ce que** la transformation est réalisée en utilisant un catalyseur en suspension de type Pd/C.

**26.** Procédé selon l'une quelconque des deux revendications précédentes, **caractérisé en ce que** la transformation est réalisée à une pression absolue dans la plage de 20 à 100 bars et à une température dans la plage de 100 à 170°C.

**27.** Procédé selon l'une quelconque des revendications 1 à 20 pour la préparation de cis-4-[3-(4-tert-butylphényl)-2-méthylpropyl]-2,6-diméthylmorpholine par transformation de Lysmeral avec de la cis-2,6-diméthylmorpholine.

**28.** Procédé selon l'une quelconque des revendications 1 à 20 pour la préparation de N,N-diméthylcyclohexylamine par transformation de cyclohexanone avec de la diméthylamine.

**29.** Procédé selon l'une quelconque des revendications 1 à 20 pour la préparation de N,N-diméthyl-N-isopropylamine par transformation d'acétone avec de la diméthylamine.

**30.** Procédé selon l'une quelconque des revendications 1 à 20 pour la préparation de dicyclohexylamine par transformation de cyclohexanone avec de la cyclohexylamine.

**31.** Procédé selon l'une quelconque des revendications 1 à 20 pour la préparation de N,N-diméthyl-N-n-propylamine par transformation de propanal avec de la diméthylamine.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3275554 A
- DE 2125039 A
- DE 3611230 A
- DE 2118283 A
- EP 7093 A1
- EP 1020424 A1
- WO 2007107477 A1
- US 4521624 A
- CN 1092061 A
- JP 10081650 A
- JP 2851274 B
- JP 02180854 A
- JP 2740828 B
- EP 611137 A
- EP 312253 A2
- EP 1312600 A
- EP 1312599 A